# EUROPEAN PATENT SPECIFICATION

(11) **EP 3 980 169 B1**
(45) Date of publication and mention of the grant of the patent: **01.05.2024**
(21) Application number: 19752564.5
(22) Date of filing: 10.06.2019
(51) Int. Cl.: C09K 23/28

(54) **EMULSIONS OBTAINED BY A BIOSUSTAINABLE PROCESS**
DURCH EIN BIONACHHALTIGES VERFAHREN ERHALTENE EMULSIONEN
ÉMULSIONS OBTENUES PAR UN PROCÉDÉ BIODURABLE

(43) Date of publication of application: 13.04.2022
(73) Proprietor: Prodotti Gianni S.r.l., 20138 Milano (IT)
(72) Inventor: ANSELMI, Cecilia, 53100 Siena (IT); CENTINI, Marisanna, 53027 San Quirico d'Orcia (SI) (IT); ROSSATO, Maria Sole, 56021 Cascina (PI) (IT); SEGA, Alessandro, 50012 Bagno a Ripoli (FI) (IT)
(74) Representative: Botti & Ferrari S.p.A.
(86) International application number: PCT/IT2019/000045
(87) International publication number: WO 2020/250250

(56) References cited:
- EP-A1- 2 476 691
- WO-A1-2013/098043
- CN-A- 106 726 660
- US-A1- 2019 133 897

## Description

### TECHNICAL FIELD

The present invention refers to the sector of surfactant emulsifiers that are used in the cosmetic, pharmaceutical and food sector. Emulsions for skin-care and hair-care with a natural connotation are described.

### BACKGROUND ART

It has long been known that surfactants have a negative environmental impact, and therefore biodegradability and biocompatibility have become important requisites, almost as much as the functionality thereof.

The increased concern about the environment has determined a greater interest in surfactants of natural origin that in the last years have become more available.

Amino acid-based surfactants have long been used with an increasing demand in the products for personal care and thanks to the excellent surface properties, wide biological activity, low toxicity and low environmental impact are a relevant alternative to conventional surfactant emulsifiers.

In the European patent application EP 2476691A1, referring to a process for obtaining peptide compounds, an α-amino acid acyl ester is disclosed as starting product to obtain a dipeptide; for example, the compound 1 (N-palmitoyl glycine ethyl ester) is synthetized by chemical synthesis from glycine ethyl ester hydrochloride in toluene added with a solution of palmitic acid chloride in toluene.

The scientific work Chemistry and Physic of Lipids (2017), 208, 43-51) concerns the molecular structure and the supramolecular organization of N-acetyl glycine alkyl esters on the basis of their acyl and alkyl chains. Various amides of the ethyl ester glycine have been obtained by reaction of the glycine ethyl ester hydrochloride in NaHCOs solution with the chloride of the acid in organic solvents.

The patent application WO 2013/098043 describes a process to generate flavours during microwave heating, wherein e.g. an amino acid is irradiated with microwaves in a suspension of sunflower oil. The patent application US 2019/133897 discloses a cream-like cosmetic cleansing composition comprising inter alia a fatty acyl amino acid. The patent application CN 106 726 660 describes a cosmetic composition comprising a fatty acyl sarcosinate.

Despite the progress in this sector, there remains the need for new emulsifiers with a completely natural connotation, having an adequate activity, with low productive cost and environmental impact, so as to increase the sanitary, economic and ethical acceptability of cosmetic, pharmaceutical and food products destined to humans or animals.

### DISCLOSURE OF INVENTION

New surfactants with emulsifying properties are described, herein called "emulsifiers" for short, amino acid-based, obtained by a green process preferably without using organic solvents, or chemically aggressive substances, said surfactants being used in the cosmetic, pharmaceutical, food sectors. The preparation process uses microwave irradiation and directly obtains the product provided with the characteristics required without the need for purification. The crude materials employed, that is, oils and/or butters and/or fatty acids, are obtained from renewable, therefore eco-friendly, sources. The process and the products respect the environment. Specifically, said emulsifiers are obtained by microwave irradiation of a mixture comprising: (a) an amino acid alkyl ester and (b) a fatty acid and/or a vegetal oil and/or butter, wherein said alkyl ester is a linear or branched C1-C10 alkyl ester. It has been surprisingly noted that such a treatment leads to a mixture of compounds provided with a significant emulsifying power, directly used for such a purpose without the need for purification. Advantageously, from the environmental and process cost point of view, the process does not require the use of any solvent: in fact, the fatty acid or the starting vegetal oil/butter already constitute a liquid means into which the reaction runs efficiently; the process does not require either the use of catalyzers (which would require a greater cost and additional passages for the removal thereof from the reaction mixture) or highly reactive substances; on the contrary, all reagents and products involved used are typically natural or natural-based, completely compatible with the environment, humans and animals. In the experimental part it is shown how to obtain emulsifiers from ethyl ester of glycine with different fatty acids (stearic acid, olive acids, oleic acid, linoleic acid) or vegetal oils (olive oil, rice bran oil, buriti oil). The resulting mixture, useful *per se* as emulsifier, comprises the amide formed between the amino acid and the fatty acids present in combination with part of the non-reacted components. The yields in amide result to be greater by using the oils. The products obtained are used to formulate emulsions, in particular of the W/O type.

### DETAILED DESCRIPTION OF THE INVENTION

For the purposes of the present invention, by "emulsifier" or "emulsifying product" is intended, as commonly known in the sector, a product that lowers the surface tension of different liquids, allowing their mutual dispersion in emulsified form; the emulsifier can be of the water in oil (W/O) or oil in water (O/W) type typically; preferably, the products of the present invention are emulsifiers of the water in oil type (W/O). The term "emulsifying product" is further used, in the present text, to indicate a composition of more than one chemical compounds, having emulsifying properties as a whole.

The terms "used as such" / "directly used" / "without purification", referred to the crude reaction mixture, are herein intended in the sense of avoiding the treatment of said mixture with any procedure of fractioning, separation, crystallization, isolation, aimed at obtaining a chemical product in its pure form; this does not exclude the performance of simple washings / filtrations in order to remove reaction slags, eventual solid material, neutralize the pH, etc.; the performance of such basic procedures remains comprised in the scope of the present invention; vice versa, the procedures of purification/isolation of pure substances herein mentioned have been conducted with the sole scope of characterizing the different components of the mixtures obtained on the basis of the present process.

Amino acid alkyl esters can be used as reagents for use in the process of the present invention, without particular limitation. It is therefore possible to use alkyl esters of: alanine, cysteine, aspartic acid, glutamic acid, aspartic acid, phenylalanine, glycine, histidine, leucine, isoleucine, lysine, methionine, asparagine, pyrrolysine, proline, glutamine, arginine, serine, threonine, selenocysteine, valine, tryptophan, tyrosine, each one of them indifferently in L-, D- or racemic form; preferred for use in the process of the invention are the non-cyclic amino acid alkyl esters; among them, particularly preferred are the alkyl esters of glycine. The alkyl ester of said amino acids is a C₁-C₁₀ alkyl ester, linear or branched, preferably ethyl or methyl esters. Preferred amino acid alkyl esters for use in the present process are ethyl or methyl ester of glycine. The amino acid alkyl ester is preferably used in the form of the corresponding hydrochloride, in the presence of sodium acetate or of an equivalent alkaline substance in molar ratio 1:1. Alternatively, it is also possible to use synthetic amino acids. The fatty acid used is an aliphatic, linear or branched long-chain monocarboxylic, saturated or unsaturated acid; typically it is a C₈-C₂₂ fatty acid, preferably C₁₆-C₁₈, such as oleic, or linoleic acid; typically it is in the liquid state at reaction temperature (as defined below) serving in such a way as reaction solvent; preferably, further, it is in the liquid state already at environmental temperature, thus allowing an easier mixing of the reagents. It is also possible to use two or more fatty acids, as defined above; an example is represented by the so-called "olive acids mixture" used herein, consisting of a mixture of the fatty acids present, in free or esterified form, in olive oil.

The vegetal oil is chosen among natural-based ones: among them we can cite, as non-limiting examples, the oils of: cashew nut, peanut, argan, avocado, buriti, coffee, safflower, coconut, canola, rice bran, corn, macadamia, almonds, walnuts, Brazil nuts, hazel nut, olive, palm, pecan, pistachio, sesame, soy, cotton seeds, sunflower seeds, pumpkin seeds, grapevine seeds, hemp, etc.; preferred for the purposes of the invention are the oils of: olive, rice bran, buriti. The present process can be conducted also using a combination of two or more vegetal oils. The choice of the oil as reagent is preferred to the use of the fatty acid: in fact, it allows to obtain generally higher yields and to incorporate in the emulsion other cosmetologically useful components, in particular natural mono-, di-, and/or triglycerides, present in the starting oil or butter, which would be more hardly incorporable in the final product if added as separate components.

The vegetal butter is chosen among natural-based ones, among them we can cite, just as non-limiting examples: cocoa butter, shea butter, cupuacu butter, olive butter, mango butter, apricot butter, murumuru butter, almonds butter, rice butter, etc.

It is also possible to use non-vegetal oils or butters, that is synthetic ones, mixed with said vegetal oils or butters; it is also possible to use synthetic triglycerides. Such an alternative use is intended to be applicable to all realizations and variants of the present invention illustrated herein.

Said amino acid alkyl ester and fatty acid can be used within wide intervals of mutual ratios. Generally, the molar ratio between amino acid alkyl ester and fatty acid is comprised between 1:1 and 1:3.

Said amino acid alkyl ester and vegetal oil and/or butter can be used within wide intervals of mutual ratios. Generally, the weight ratio between amino acid alkyl ester and vegetal oil and/or butter is comprised between 0.2:1 and 1:1.

In performing the process according to the invention, said amino acid alkyl ester and fatty acid or vegetal oil and/or butter are placed in contact, and preferably mixed, in an appropriate reactor. The mixture of reagents is then subjected to microwave irradiation, under agitation; the irradiation is performed so as to heat the reaction mixture at a temperature comprised between 80 and 120°C, for an overall period of time (intended as residence time of the mixture at said temperature) comprised between 10 and 40 minutes, for example of 15, 20 or 30 minutes, under environmental pressure. The reaction leads to the formation of an amide between the amino group of the amino acid alkyl ester and the acyl group of a fatty acid: the acyl in question is that constituting the used fatty acid or (in the process variant using the vegetal oil and/or butter) is that constituting a fatty acid contained, in free or esterified form as triglyceride, in the used vegetal oil or butter.

In order to increase the reaction yield (intended as percentage of formation of amide of the amino acid alkyl ester) it is possible to perform the process by subjecting the resulting mixture to one or more further irradiation cycles, for example 2 or 3 cycles, under said conditions; particularly high yields have been obtained through 2 process cycles of 30 minutes each, at 120°C. Alternatively, or in addition to the application of further cycles, it is also possible to perform further recycles: in this case the reaction mixture is first added again with amino acid alkyl ester and then subjected to further irradiation under the conditions mentioned above; such a procedure is repeated on the basis of the intended number of recycles.

The maximization of the yield is not the objective of the present invention; in fact, it has been discovered that the incomplete reaction product, comprising said amide in mixture with the non-reacted acid or oil or butter and a minor portion (≤ 5% by weight on the reaction product, e.g. between 0.5 and 5%) of a dipeptide of formula (I) 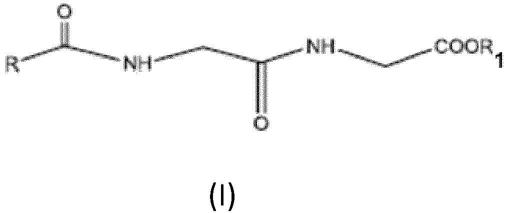 where R represents the aliphatic chain of the fatty acid used (or present in the vegetal oil or butter used) and R₁ represents the alkyl group of the amino acid alkyl ester involved in the reaction, shows a useful emulsifying effect and can be directly used as such for this purpose. The emulsifying effect is particularly consistent and stable for the reaction products, intended as a crude reaction mixture, which contain at least the 8% by weight of amide of the amino acid alkyl ester; in case of use of vegetal oils or butters, said percentage of amide is intended to be calculated with reference to the amide with the fatty acid present in highest quantity in the oil or butter used; when using vegetal oils or butters, the yield is intended to be calculated with reference to the sum of the respective amides. For that purpose, it is possible to analyze the mixture resulting from the first reaction and, in case of weight percentages of amide lower than the 8%, subject the mixture to further cycles or recycles as indicated above, until reaching the desired percentage. Particularly efficient percentages of amide are comprised between 8 and 40% and eventually also ≤ 100%.

A further object of the present invention is the emulsifying product that can be obtained by the process described above, i.e. by microwave irradiation of a mixture comprising: (a) an amino acid alkyl ester and (b) a fatty acid or a vegetal oil or butter. Such a product is typically a crude reaction product, eventually treated only by basic washings/filtrations in order to eliminate undesired slag and/or bring the pH to neutrality. Said product is advantageously characterized by being the result of a highly eco-compatible process, i.e. free from substances that compromise the use thereof in products for humans or animals. It is further characterized by a low production cost, being obtained by a simple process with respect to the modality of execution and the limited number of steps, with a low energetic demand and without the use of expensive reagents: consequently, the use of this product as emulsifying additive allows the preparation of emulsifying products for the skin and the hair (e.g. cosmetic emulsions such as creams, lotions, detergents, conditioners, make-up, pharmaceutical emulsions, etc.) having lower costs with respect to those currently available. This further contributes to the preparation of highly eco-friendly products, increasingly appreciated by consumers. In said emulsifying product, alternatively or mixed with said vegetal oils or butters, it is also possible to use non vegetal oils or butters, that is, synthetic ones; it is also possible to use synthetic triglycerides. Such an alternative use is intended to be applicable to all embodiments and variants of the present invention illustrated herein.

The product that is the object of the invention can also be characterized independently from the productive process, as a natural emulsifying product, liquid under environmental conditions, free from organic solvents, comprising in a mixed and homogeneously dispersed form:
(a) an amino acid alkyl ester
(b) a fatty acid or a vegetal oil or butter
(c) at least the 8% by weight of said amino acid alkyl ester in the form of amide with a fatty acid;
(d) 5% or less by weight of a compound of formula (I): 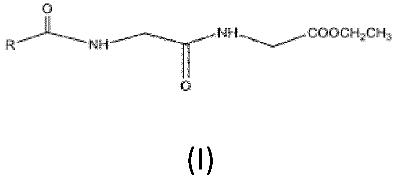
wherein R is the alkyl radical of the fatty acid referred in (b) or of a fatty acid contained in the vegetal oil or butter referred in (b).

In such a variant, the amino acid alkyl ester, the fatty acid, the vegetal oil or butter and the amide are further intended to be characterized on the basis of one or more of the sub-features previously illustrated with relation to the present process.

A further object of the invention is the use of the product as described above as emulsifying additive, in particular in the cosmetic, pharmaceutical and/or food sector. A further object of the invention is an emulsified product for cosmetic, pharmaceutical and/or food use comprising, as emulsifying additive, the emulsifying product described herein. The emulsifiers described herein are particularly useful in the skin-care, hair-care and make-up fields. A particularly preferred product is represented by sun and anti-aging creams. In fact, it has been seen that the emulsifying system in accordance with the invention, in addition to mainly functioning as emulsifier, also increases the sun protector factor, thus conferring an unexpected "booster effect" to the protection conferred by the cream.

With the present invention a completely natural emulsifying product has therefore been obtained, with a low environmental impact based on microwave irradiation, useful in the cosmetic, pharmaceutical and food sectors. The invention is further described hereinafter in a non-limiting manner by the following experimental examples.

### EXAMPLES

### 1. Materials and methods

The ethyl ester of glycine has been chosen as working example with the purpose of obtaining emulsifiers, in particular of the W/O type. The synthesis free from solvents has been selected in order to realize reactions according to the principles of the green Chemistry with microwave irradiation. In this process the reagents have been used as such and introduced in the reaction container without the use of organic solvents. The yields of the reaction have been assessed as percentage of amide in the crude reaction product after washing with water.

The yields and the differences in yield have been assessed by using as reagent the fatty acids or, alternatively, the vegetal oils. In the second case, the yields were found to be generally greater and in the reaction mixture also non-reacted oil, mono and di-glycerides have been found.

The conditions of the reaction have been further varied by monitoring the yields: the attention has been focused on time and number of microwave irradiation cycles as well as on recycle of the reaction mixture. The influence of using the amino acid alkyl ester in form of hydrochloride in presence of sodium acetate, has also been assessed.

### 2. Treatment and results

The results of the tests done with oils are presented in Figure 1, where in ordinate are shown the yields (percentage of amide on the crude reaction product) and in abscissa are shown the different methods of treatment with microwaves.

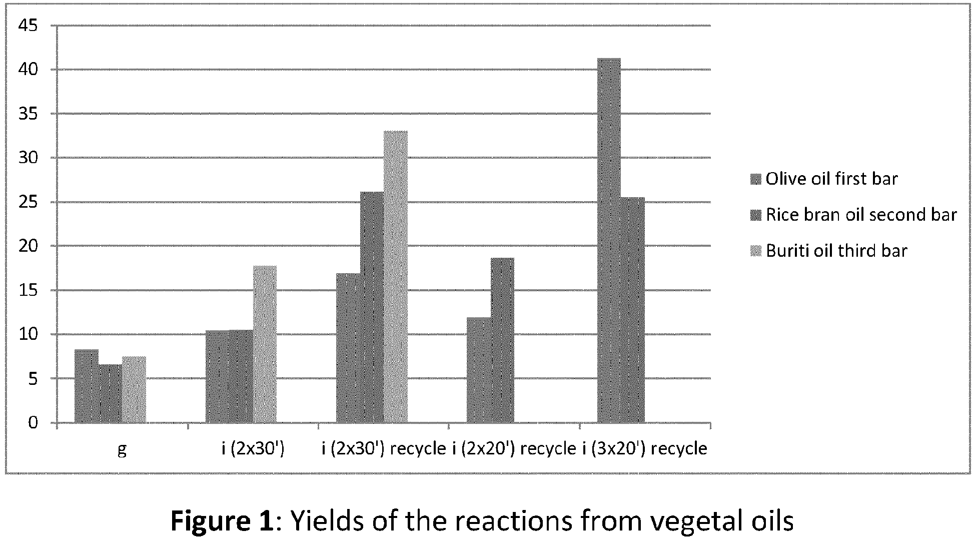

### Method g:

- Temperature 120°C
- Oil: Amino Acid Ester ratio= 5:1 (that is, 1:0,2) by weight
- Microwave irradiation (MW): 60' (two cycles of 30' each)

### Method i:

- Temperature 120°C
- Oil: Amino Acid Ester (hydrochloride) ratio = 5:1 by weight
- Amino Acid Ester (hydrochloride): Sodium Acetate ratio = 1:1 molar
- Microwave irradiation: 60' (two cycles of 30' each)

| **Oil used** | **Synthesis method MW irr.** | **Yield %** | **Analysis method** | **Aspect of the product** |
|---|---|---|---|---|
| Olive oil | **i**, 2x30 min recycles | 16.91 | HPLC | Yellow-orange liquid |
| Rice bran oil | **i**, 2x30 min recycles | 26.15 | HPLC | Orange liquid |
| Buriti oil | **i**, 2x30 min recycles | 33.11 | HPLC | Red liquid |
| Olive oil | **i**_{,} 10 min | 6.36 | HPLC | Yellow-orange liquid |
| Olive oil | **i**, 20 min | 8.40 | CC* | Yellow-orange liquid |
| | | 7.76 | HPLC | |
| Olive oil | **i**, 30 min | 11.94 | CC | Yellow-orange liquid |
| | | 9.15 | HPLC | |
| Olive oil | **i**, 2x20 min recycles | 11.90 | HPLC | Yellow-orange liquid |
| Rice bran oil | **i**, 2x20 min recycles | 18.67 | HPLC | Orange liquid |
| Rice bran oil | **i**, 3x20 min recycles | 25.50 | HPLC | Orange liquid |
| Olive oil | **i**, 3x20 min recycles | 41.30 | HPLC | Yellow-orange liquid |

| | | | | |
|---|---|---|---|---|
| *CC: Column Chromatography | | | | |

The purification has allowed the separation of a secondary product (dipeptide by-product) characterized by NMR, of formula (I) where R corresponds to the alkyl radical of the fatty acid.

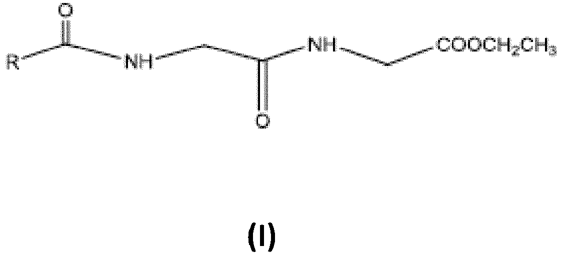

### Characterization:

The reaction products both as crude materials (after washing with water) and as pure products (after purification with Layer or Column Chromatography) were analyzed by ¹H-NMR.

The HPLC analysis has allowed the identification and the quantification of the oleic and linoleic acids. The differences in the yields of the two amides reflect the percentage of the acid components in the starting oils. The synthetized compounds have lipophilic characteristics.

**Table 1: Chemical shifts of some of the pure amides obtained with the fatty acids and with the oils**

| **PRODUCT** | **1H NMR δ** |
|---|---|
| *Pure amide from fatty acids of olive oil* | 0,85 (t, 3H, -CH₃); 1,18-1,40 (bp, nH, -(CH₂)ₙ e - OCH₂CH₃); 1,62 (bp, 2H, -COCH₂CH₂-); 2,01 (m, 4H, - CH₂CH=CHCH₂-); 2,22 (t, 2H,-HNCOCH₂-); 2,76 (t, 2H, - CH=CHCH₂CH=CH-); 4,02 (d, 2H, -OCOCH₂NHCO-); 4,22 (q, 2H, -OCH₂CH₃); 5,33 (bp, 2H, -CH=CH-); 5,91 (bp, 1H, NH). |
| *Pure amide from oleic acid* | 0,86 (t, 3H, -CH₃); 1,23-1,29 (bp, nH, -(CH₂)ₙ e - OCH₂CH₃); 1,61 (bp, 2H, -COCH₂CH₂-); 1,99 (m, 4H, - CH₂CH=CHCH₂-); 2,21 (t, 2H, -HNCOCH₂-); 2,75 (t, 2H, - CH=CHCH₂CH=CH-); 4,02 (d, 2H, -OCOCH₂NHCO-); 4,20 (q, 2H, -OCH₂CH₃); 5,31 (bp, 2H, -CH=CH-); 5,89 (bp, 1H, NH). |
| *Pure amide from olive oil* | 0,86 (t, 3H, -CH₃); 1,15-1,40 (bp, nH, -(CH₂)ₙ e - OCH₂CH₃); 1,61 (bp, 2H, -COCH₂CH₂); 1,99 (m, 4H, - CH₂CH=CHCH₂-); 2,21 (t, 2H, -HNCOCH₂-); 2,75 (t, 2H, -CH=CHCH₂CH=CH-); 4,02 (d, 2H, -OCOCH₂NHCO-); 4,21 (q, 2H, -OCH₂CH₃); 5,35 (bp, 2H, -CH=CH-); 5,90 (bp, 1H, NH). |
| *Pure amide from rice bran oil* | 0,86 (t, 3H, -CH₃); 1,23-1,27 (bp, nH, -(CH₂)ₙ e - OCH₂CH₃); 1,61 (bp, 2H, -COCH₂CH₂-); 1,99 (m, 4H, - CH₂CH=CHCH₂-); 2,21 (t, 2H, -HNCOCH₂-); 2,74 (t, 2H, -CH=CHCH₂CH=CH-); 4,00 (d, 2H, -OCOCH₂NHCO-); 4,19 (q, 2H, -OCH₂CH₃); 5,32 (bp, 2H, -CH=CH-); 5,97 (bp, 1H, NH). |
| *Pure amide from coffee oil* | 0,87 (t, 3H, -CH₃); 1,23-1,29 (bp, nH, -(CH₂)ₙ e - OCH₂CH₃); 1,62 (bp, 2H, -COCH₂CH₂-); 2,02 (m, 4H, - CH₂CH=CHCH₂-); 2,21 (t, 2H, -HNCOCH₂-); 2,75 (t, 2H, -CH=CHCH₂CH=CH-); 4,02 (d, 2H, -OCOCH₂NHCO-); 4,20 (q, 2H, -OCH₂CH₃); 5,33 (bp, 2H, -CH=CH-); 5,90 (bp, 1H, NH). |

**Table 2: Chemical shifts of the amide (by product) obtained from the oleic acid**

| **PRODUCT** | **MOLECULAR STRUCTURE** |
|---|---|
| *Second amide from olive oil* | 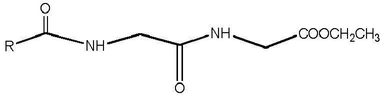 |

| **1H NMR δ** | |
|---|---|
| 0,87 (t, 3H, -CH₃); 1,23-1,29 (bp, nH, -(CH₂)ₙ e -OCH₂CH₃); 1,62 (bp, 2H, -COCH₂CH₂-); 2,02 (m, 4H, -CH₂CH=CHCH₂-); 2,22 (t, 2H, -HNCOCH₂-); 2,31 (t, 2H, -OHCOCH₂-); 2,75 (t, 2H, - CH=CHCH₂CH=CH-); 3,97 (d, 2H, -CONHCH₂CONHCH₂COO-); 4,02 (d, 2H, - CONHCH₂CONHCH₂COO-); 5,33 (bp, 2H, -CH=CH-); 6,12 (bp, 1H, -CONHCH₂CONHCH₂COO-); 6,43 (bp, 1H, -CONHCH₂CONHCH₂COO-). | |

**Table 3: Chemical shifts of the crude products after washing with water**

| **PRODUCT** | **1H NMR δ** |
|---|---|
| *Crude product from olive fatty acids* | 0,85 (t, 3H, -CH₃); 1,18-1,40 (bp, nH, -(CH₂)ₙ e -OCH₂CH₃); 1,57 (bp, 2H, -COCH₂CH₂-); 1,96 (m, 4H, -CH₂CH=CHCH₂-); 2,25 (t, 2H,-HNCOCH₂-): 2,74 (t, 2H, - CH=CHCH₂CH=CH-); 3,69 (bp, 2H, -OCOCH₂NH₂) 4,01 (d, 2H, -OCOCH₂NHCO-); 4,19 (q, 2H, -OCH₂CH₃); 5,31 (bp, 2H, -CH=CH-). |
| *Crude product from oleic acid* | 0,85 (t, 3H, -CH₃); 1,24-1,27 (bp, nH, -(CH₂)ₙ e -OCH₂CH₃); 1,57 (bp, 2H, -COCH₂CH₂-); 1,97 (m, 4H, -CH₂CH=CHCH₂-); 2,26 (t, 2H, -HNCOCH₂-): 2,74 (t, 2H, - CH=CHCH₂CH=CH-); 3,68 (m, 2H, -OCOCH₂NH₂); 4,01 (d, 2H, -OCOCH₂NHCO-); 4,19 (q, 2H, -OCH₂CH₃); 5,31 (bp, 2H, -CH=CH-); 9,26 (s, 1H, -COOH). |
| *Crude product from linoleic acid* | 0,59 (t, 3H, -CH₃); 1,19-1,27 (bp, nH, -(CH₂)ₙ e -OCH₂CH₃); 1,55 (bp, 2H, -COCH₂CH₂-);1,94 (bp, 2H, -COCH₂CH₂-) 2,02 (m, 4H, -CH₂CH=CHCH₂-); 2,25 (t, 2H, -HNCOCH₂-): 2,74 (t, 2H, -CH=CHCH₂CH=CH-); 3,70 (q, 2H, -OCH₂CH₃); 4,00 (d, 2H, -OCOCH₂NHCO-); 4,20 (q, 2H, -OCH₂CH₃); 5,32 (bp, 2H, -CH=CH-). |
| *Crude product from stearic acid* | 0,86 (t, 3H, -CH₃); 1,15-1,32 (bp, nH, -(CH₂)ₙ e -OCH₂CH₃); 1,61 (bp, 2H, -COCH₂CH₂-); 2,22 (t, 2H, -HNCOCH₂-); 2,33 (t, 2H, -OHCOCH₂-); 3,97 (d, 2H, -CH₂NHCOCH₂NHCO-); 4,02 (d, 2H, -OCOCH₂NHCO-); 4,21 (q, 2H, -OCH₂CH₃); 5,90 (bp, 1H, NH); 6,12 (bp, 1H, NH). |
| *Crude product from olive oil* | 0,85 (t, 3H, 3X-CH₃); 1,18-1,38 (bp, nH, -(CH₂)ₙ e - OCH₂CH₃); 1,60 (bp, 2H, -COCH₂CH₂-); 1,98 (m, 4H, - CH₂CH=CHCH₂-); 2,22 (t, 2H, -HNCOCH₂-); 2,30 (t, 2H, - OHCOCH₂-); 2,74 (t, 2H, -CH=CHCH₂CH=CH-); 3,70 (d, 2H, -CH₂OH); 4,01 (d, 2H, -OCOCH₂NHCO-); 4,13-4,27 (dd, 4H, -CH₂OCHOCH₂O-); 4,20 (q, 2H, -OCH₂CH₃); 5,23 (m, 1H, CH₂OCHOCH₂O-); 5,31 (m, 2H, -CH=CH-); 5,95 (bp, 1H, -NH-). |
| *Crude product from rice bran oil* | 0,83 (t, 3H, 3X-CH₃); 1,18-1,38 (bp, nH, -(CH₂)ₙ e - OCH₂CH₃); 1,57 (bp, 2H, -COCH₂CH₂-); 1,98 (bp, 4H, - CH₂CH=CHCH₂-); 2,21 (t, 2H, -HNCOCH₂-); 2,28 (t, 2H, - OHCOCH₂-); 2,74 (t, 2H, -CH=CHCH₂CH=CH-); 3,70 (d, 2H, -CH₂OH); 3,99 (d, 2H, -OCOCH₂NHCO-); 4,11-4,27 (dd, 4H, -CH₂OCHOCH₂O-); 4,16 (q, 2H, -OCH₂CH₃); 5,22 (m, 1H, CH₂OCHOCH₂O-); 5,30 (m, 2H, -CH=CH-); 6,01 (bp, 1H, -NH-). |
| *Crude product from buriti oil* | 0,85 (t, 3H, 3X-CH₃); 1,28 (bp, 2H, -(CH₂-), e -OCH₂CH₃); 1,60 (bp, 2H, -COCH₂CH₂-); 2,00 (m, 4H, - CH₂CH=CHCH₂-); 2,20 (t, 2H, -HNCOCH₂-); 2,29 (t, 2H, - OHCOCH₂-); 2,73 (t, 2H, -CH=CHCH₂CH=CH-); 3,70(d, 2H, -CH₂OH); 4,00 (d, 2H, -OCOCH₂NHCO-); 4,05-4,15 (dd, q, 4H, 2H -CH₂OCHOCH₂O-, -OCH₂CH₃); 4,21 (q, 2H, -OCH₂CH₃); 5,22 (m, 1H, -CH₂OCHOCH₂O-); 5,32 (m, 2H, -CH=CH-); 6,05 (bp, 1H, NH). |
| *Crude product from coffee oil* | 0,80 (t, 3H, 3X-CH₃); 1,12-1,36 (bp, nH, -(CH₂)ₙ e - OCH₂CH₃); 1,54 (bp, 2H, -COCH₂CH₂-); 1,98 (bp, 4H, - CH₂CH=CHCH₂-); 2,20 (t, 2H, -HNCOCH₂-); 2,24 (t, 2H, - OHCOCH₂-); 2,71 (t, 2H, -CH=CHCH₂CH=CH-); 3,70 (d, 2H, -CH₂OH); 3,95 (d, 2H,-OCOCH₂NHCO-); 4,08-4,23 (dd, 4H, -CH₂OCHOCH₂O-); 4,15 (q, 2H, -OCH₂CH₃); 5,21 (m, 1H, CH₂OCHOCH₂O-); 5,28 (m, 2H, -CH=CH-); 6,12 (bp, 1H, NH). |

### 3. Emulsification tests

### 3.1 Basis emulsion

A basis formulation has been used for testing the crude products obtained by the synthesis from the acids or from the oils. Such a basis formulation has been added with the emulsifying mixture object of the invention obtained by reaction of the ethyl ester of glycine, alternatively with fatty acids or oils.

| **Basis emulsion** | | | |
|---|---|---|---|
| | ***COMMERCIAL NAME*** | ***INCI NAME*** | ***QUANTITY*** |
| 1 | EMULSIFYING MIXTURE | | 5% |
| 2 | CERA BELLINA | *Polyglyceryl-3 beeswax* | 5% |
| 3 | STRINGY WHITE VASELINE | *Petrolatum* | 5% |
| 4 | VASELINE OIL | *Paraffinum liquidum* | 25% |
| 5 | WATER | *Aqua* | q.s. at 100 |
| 6 | GLYCERINE | *Glycerin* | 4,5% |
| 7 | MAGNESIUM SULPHATE | *Magnesium sulfate* | 0,5% |
| 8 | APEROXID TLA | *Tocopherol, Lecithin, Ascorbyl palmitate* | 0,05% |

### 3.2 Amino acid amide-based W/O emulsions obtained with acids

The emulsifying properties of the products obtained from the reaction of the acids with the amino acid have been assessed (Table 4).

The emulsions have undergone the following tests: viscosity, organoleptic properties, stability with accelerated ageing (centrifugation and different temperature conditions 5-25-40°C and thermal stress 5/40°C for three months.

**Table 4: aspect and stability at 25°C of the emulsions prepared with the mixtures obtained from fatty acids.**

| **FATTY ACID** | **PERCENTAGE OF AMIDE** | **ASPECT OF THE EMULSION** | **STABILITY** |
|---|---|---|---|
| **REFERENCE AMIDE (*)** | 100 | White, homogeneous | 3 months |
| **MIXTURE OF OLIVE ACIDS** | 9,11 | White, consistent | 1 month |
| **OLEIC ACID** | 12,13 | White, consistent | 2 months |
| **LINOLEIC ACID** | 4,38 | It forms but it separates immediately | Unstable |
| **STEARIC ACID** | 6,37 | White, consistent waxy | Unstable |

| | | | |
|---|---|---|---|
| *(*) amide of glycine ethyl ester with mixture of fatty acids of olive oil* | | | |

The stability of the emulsion increases on the basis of the percentage of amide present in the reaction product.

### 3.3 Amino acid amide-based W/O emulsions obtained with vegetal oils

The same procedure described above has been applied to the use of products obtained from vegetal oils. The substances obtained as described by microwave irradiation can be used as such. They contain the amide and also mono and di-glycerides of the fatty acids and non-reacted oil, which can be used in cosmetic emulsions.

**Table 5**

| **OIL** | **PERCENTAGE OF AMIDE** | **ASPECT OF THE EMULSION** | **STABILITY** |
|---|---|---|---|
| **OLIVE OIL** | 8,07 | Cream color, fluid | 2 months |
| **OLIVE OIL** | 16,91 | White, consistent | 3 months |
| **RICE BRAN OIL** | 10,49 | Cream color, consistent | 2 months |
| **RICE BRAN OIL** | 26,15 | White, consistent | 3 months |
| **BURITI OIL** | 17,75 | Yellow, consistent | 3 months |
| **BURITI OIL** | 33,11 | Light yellow, consistent | 3 months |

The stability of the emulsion increases on the basis of the percentage of amide present in the reaction product.

### Sunscreen emulsions

In this experiment, the crude reaction product between glycine ethyl ester and vegetal oils (olive oil, rice bran oil and buriti oil) has been used as emulsifier in sunscreen emulsions containing two UVB (*Ethylhexyl Methoxycinnamate*) and UVA (*Butylmethoxy dibenzoyl Methane*) chemical filters. Sunscreen emulsions have been prepared on the basis of the following composition, using as emulsifier the crude reaction product according to the invention or (for the reference composition) a standard emulsifier derived from olive oil (Sorbitan Olivate).

| **Sunscreen emulsion with OLIVEM 900 (reference)** | | | |
|---|---|---|---|
| | ***COMMERCIAL NAME*** | ***INCI NAME*** | ***QUANTITY*** |
| 1 | **OLIVEM 900** | *Sorbitan olivate* | 7,5% |
| 2 | CERA BELLINA | *Polyglyceryl-3 beeswax* | 5% |
| 3 | STRINGY WHITE VASELINE | *Petrolatum* | 5% |
| 4 | VASELINE OIL | *Paraffinum liquidum* | 20% |
| 5 | **EUSOLEX 2292** | *Ethylhexylmethoxycinnamate* | 6,5% |
| 6 | **EUSOLEX 9020** | *Butylmethyoxydibenzoylmethane* | 3,5% |
| 7 | AQUA | *Aqua* | q.s. at 100 |
| 8 | GLYCERINE | *Glycerin* | 4,5% |
| 9 | MAGNESIUM SUPLHATE | *Magnesium sulfate* | 0,5% |

| **Sunscreen emulsion** | | | |
|---|---|---|---|
| | ***COMMERCIAL NAME*** | ***INCI NAME*** | ***QUANTITY*** |
| 1 | **EMULSIFYING MIXTURE** | | **5%** |
| 2 | CERA BELLINA | *Polyglyceryl-3 beeswax* | 5% |
| 3 | STRINGY WHITE VASELINE | *Petrolatum* | 5% |
| 4 | VASELINE OIL | *Paraffinum liquidum* | 25% |
| 5 | **EUSOLEX 2292** | *Ethylhexylmethoxycinnamate* | 6,5% |
| 6 | **EUSOLEX 9020** | *Butylmethyoxydibenzoylmethane* | 3,5% |
| 7 | AQUA | *Aqua* | q.s. at 100 |
| 8 | GLYCERINE | *Glycerin* | 4,5% |
| 9 | MAGNESIUM SUPLHATE | *Magnesium sulfate* | 0,5% |
| 10 | APEROXID TLA | *Tocopherol, Lecithin, Ascorbyl palmitate* | 0,05% |

All the emulsions have resulted to be sterile and have been tested to assess the SPF (Sun Protection Factor) thereof in vitro. The results are shown in Table 6.

**Table 6**

| **EMULSIFIER** | **SPF** |
|---|---|
| **SORBITAN OLIVATE** | 22 |
| **DERIVED FROM OLIVE OIL** | 24 |
| **DERIVED FROM RICE BRAN OIL** | 28 |
| **DERIVED FROM BURITI OIL** | 26 |

The SPF values show an evident SPF Booster due to the emulsifying system. This effect could be attributed to the better rheological properties of the amide-based emulsions that have better spreading properties with respect to those obtained with the reference emulsifier, as well as to the content of antiradical substances contained in the oils and in their unsaponifiable fraction.

## Claims

1. Process for the production of an emulsifying composition, comprising irradiating by microwaves a mixture comprising: (a) an amino acid alkyl ester and (b) a fatty acid and/or a vegetal oil and/or butter, wherein said alkyl ester is a linear or branched C₁-C₁₀ alkyl ester.

2. Process according to claim 1, wherein said alkyl ester is an ethyl ester.

3. Process according to claims 1-2, wherein said amino acid is glycine.

4. Process according to claims 1-3, wherein said fatty acid is oleic acid.

5. Process according to claims 1-3, wherein said vegetal oil is olive oil, rice bran oil, buriti oil or a mixture thereof.

6. Process according to claims 1-5, wherein said irradiating by microwaves comprises heating said mixture at a temperature comprised between 80 and 120°C for a period of time comprised between 10 and 40 minutes, at environmental pressure.

7. Process according to claims 1-6, wherein the reaction product is retreated as described in claims 1-6, for a number of further cycles comprised between 2 and 5.

8. Process according to claims 1-7, wherein instead of vegetal oil and/or butter a synthetic oil and/or butter is used.

9. Emulsifying product obtained by the process according to claims 1-8.

10. Natural emulsifying composition, liquid at environmental conditions, comprising in a mixed and homogeneously dispersed form:
(a) an amino acid alkyl ester
(b) a fatty acid liquid at environmental temperature and/or vegetal oil
(c) at least 8% by weight of said amino acid alkyl ester whose amino group is in the form of amide;
(d) 5% or less by weight of a compound of formula (I): 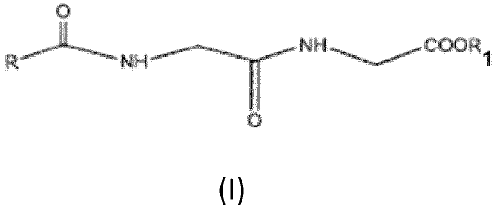 wherein R represents the alkyl radical of the fatty acid (or of a fatty acid present in the vegetal oil) referred in (b) and R₁ represents the alkyl radical of the alkyl ester referred in (a).

11. Use of the product or composition according to claims 9 or 10 as emulsifying agent.

12. Cosmetic, pharmaceutical or food composition comprising, as emulsifying agent, the product or composition according to claims 9 or 10, preferably at a weight/weight concentration comprised between 1 and 10%.

13. Composition according to claim 12, being a water in oil emulsion (W/O).

14. Composition according to claims 12-13, in the form of sunscreen cream, with protective and anti-aging (make-up) features.

## Patentansprüche

1. Verfahren zur Herstellung einer emulgierenden Zusammensetzung, umfassend mit Mikrowellen Bestrahlen eines Gemisches, umfassend: (a) einen Aminosäurealkylester und (b) eine Fettsäure und/oder ein pflanzliches Öl und/oder Butter, wobei der Alkylester ein linearer oder verzweigter C₁-C₁₀-Alkylester ist.

2. Verfahren nach Anspruch 1, wobei der Alkylester ein Ethylester ist.

3. Verfahren nach Anspruch 1-2, wobei die Aminosäure Glycin ist.

4. Verfahren nach Anspruch 1-3, wobei die Fettsäure Oleinsäure ist.

5. Verfahren nach Anspruch 1-3, wobei das pflanzliche Öl Olivenöl, Reiskleieöl, Buriti-ÖI oder ein Gemisch davon ist.

6. Verfahren nach Anspruch 1-5, wobei das mit Mikrowellen Bestrahlen Erhitzen des Gemisches bei einer Temperatur, die zwischen 80 und 120°C liegt, für einen Zeitraum, der zwischen 10 und 40 Minuten liegt, bei Umgebungsdruck umfasst.

7. Verfahren nach Anspruch 1-6, wobei das Reaktionsprodukt wie in Anspruch 1-6 beschrieben für eine Anzahl von weiteren Zyklen, die zwischen 2 und 5 liegen, behandelt wird.

8. Verfahren nach Anspruch 1-7, wobei anstelle von pflanzlichem Öl und/oder Butter ein synthetisches Öl und/oder Butter verwendet wird.

9. Emulgierendes Produkt, das nach dem Verfahren gemäß den Ansprüchen 1-8 erhalten wird.

10. Natürliche emulgierende Zusammensetzung, flüssig unter Umgebungsbedingungen, die in gemischter und homogen dispergierter Form umfasst:
(a) einen Aminosäurealkylester
(b) eine bei Umgebungstemperatur flüssige Fettsäure und/oder ein pflanzliches Öl
(c) mindestens 8 Gew.-% des Aminosäurealkylesters, dessen Aminogruppe in Form eines Amids vorliegt;
(d) 5 Gew.-% oder weniger einer Verbindung der Formel (I): 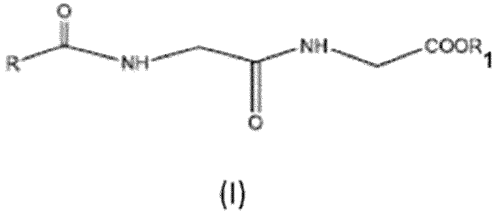
wobei R den Alkylrest der Fettsäure (oder einer in dem Pflanzenöl vorhandenen Fettsäure) gemäß (b) darstellt und R₁ den Alkylrest des Alkylesters gemäß (a) darstellt.

11. Verwendung des Produkts oder der Zusammensetzung nach Anspruch 9 oder 10 als emulgierendes Mittel.

12. Kosmetische, pharmazeutische oder Nahrungsmittelzusammensetzung, die als emulgierendes Mittel das Produkt oder die Zusammensetzung nach Anspruch 9 oder 10 umfasst, vorzugsweise in einer Gewicht/Gewicht-Konzentration, die zwischen 1 und 10% umfasst.

13. Zusammensetzung nach Anspruch 12, die eine Wasser-in-Öl-Emulsion (W/O) ist.

14. Zusammensetzung nach den Ansprüchen 12-13, in Form einer Sonnencreme, mit Schutz- und Anti-Aging (Make-up) Eigenschaften.

## Revendications

1. Procédé pour la production d'une composition émulsionnante, comportant l'irradiation par micro-ondes d'un mélange comportant : (a) un ester d'alkyle d'acide aminé et (b) un acide gras et/ou une huile végétale et/ou du beurre, dans lequel ledit ester d'alkyle est un ester d'alkyle en C₁ à C₁₀ linéaire ou ramifié.

2. Procédé selon la revendication 1, dans lequel ledit ester d'alkyle est un ester d'éthyle.

3. Procédé selon les revendications 1 à 2, dans lequel ledit acide aminé est la glycine.

4. Procédé selon les revendications 1 à 3, dans lequel ledit acide gras est l'acide oléique.

5. Procédé selon les revendications 1 à 3, dans lequel ladite huile végétale est l'huile d'olive, l'huile de son de riz, l'huile de buriti ou un mélange de celles-ci.

6. Procédé selon les revendications 1 à 5, dans lequel ladite irradiation par micro-ondes comporte le chauffage dudit mélange à une température comprise entre 80 et 120 °C pendant une période de temps comprise entre 10 et 40 minutes, à pression ambiante.

7. Procédé selon les revendications 1 à 6, dans lequel le produit de réaction est retraité tel que décrit dans les revendications 1 à 6 pendant un nombre de cycles supplémentaires compris entre 2 et 5.

8. Procédé selon les revendications 1 à 7, dans lequel à la place d'huile végétale et/ou de beurre, une huile synthétique et/ou du beurre est utilisé.

9. Produit émulsionnant obtenu par le procédé selon les revendications 1 à 8.

10. Composition émulsionnante naturelle, liquide aux conditions ambiantes, comportant une forme mélangée et dispersée de façon homogène :
(a) un ester d'alkyle d'acide aminé,
(b) un acide gras liquide à température ambiante et/ou une huile végétale,
(c) au moins 8 % en poids dudit ester d'alkyle d'acide aminé dont le groupe amino se présente sous la forme d'un amide,
(d) 5 % ou moins en poids d'un composé de formule (I) : 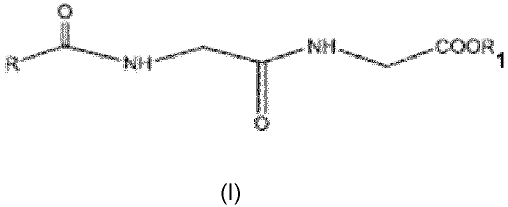
dans laquelle R représente le radical alkyle de l'acide gras (ou d'un acide gras présent dans l'huile végétale) cité en (b) et R₁ représente le radical alkyle de l'ester d'alkyle cité en (a).

11. Utilisation du produit ou de la composition selon les revendications 9 ou 10 en tant qu'agent émulsionnant.

12. Composition cosmétique, pharmaceutique ou alimentaire comportant, en tant qu'agent émulsionnant, le produit ou la composition selon les revendications 9 ou 10, de préférence à une concentration poids/poids comprise entre 1 et 10 %.

13. Composition selon la revendication 12, étant une émulsion eau dans huile (W/O).

14. Composition selon les revendications 12 à 13, ayant la forme d'une crème de protection solaire, avec des caractéristiques de protection et d'antivieillissement (maquillage).
